# EUROPEAN PATENT SPECIFICATION

(11) **EP 2 026 853 B1**
(45) Date of publication and mention of the grant of the patent: **09.09.2009**
(21) Application number: 07845258.8
(22) Date of filing: 16.05.2007
(51) Int. Cl.: A61L 27/28, A61L 27/30, A61L 27/54, A61L 31/08, A61F 2/82, A61C 8/00

(54) **ANISOTROPIC NANOPOROUS COATINGS FOR MEDICAL IMPLANTS**
ANISOTROPE NANOPORÖSE BESCHICHTUNGEN FÜR MEDIZINISCHE IMPLANTATE
COUCHES ANISOTROPIQUES ET NANOPOREUSES POUR DES IMPLANTES MÉDICAUX

(30) Priority: 17.05.2006 EP 06114127; 07.08.2006 EP 06118544
(43) Date of publication of application: 25.02.2009
(73) Proprietor: DEBIOTECH S.A., 1004 Lausanne (CH)
(72) Inventor: PIVETEAU, Laurent-Dominique, 1030 Bussigny (CH); HOFMANN, Heinrich, 1009 Pully (CH); NEFTEL, Frédéric, 1005 Lausanne (CH)
(74) Representative: Grosfillier, Philippe
(86) International application number: PCT/IB2007/051869
(87) International publication number: WO 2007/148240

(56) References cited:
- EP-A1- 1 319 416
- WO-A-98/28025
- WO-A2-20/04043292
- US-B1- 6 709 379
- "Debiotech S.A. announced today a collaborative alliance with the Ecole Polytechnique Fédérale de Lausanne, Switzerland, in the field of drug-eluting coatings" [Online] 14 February 2006 (2006-02-14), DEBIOTECH , XP002412996 Retrieved from the Internet: URL:www.debiotech.com/news/nw_158.html> [retrieved on 2007-01-02] the whole document

## Description

The present invention relates to a process for fabricating porous coatings with controlled structure in the micro and nano-size domain. In particular, but not exclusively, it relates to a process for fabricating coatings with an anisotropic pore size distribution and to coatings obtained using such coatings.
It also relates to porous coatings with controlled structure in the micro and nano-size domain. The coatings have a thickness between 10 nanometers and 10 millimeters and their porosity is created in such a way that the pore size distribution is anisotropic.
The invention finally relates objects covered with said coatings.

### State of the art

Coatings may be used in a great.variety of technical field, in particular in the medical field.
Failure of a therapy based on systemically administered drugs has many origins, but one classical reason is the inability to achieve the required dose at the site to be treated. This effect is especially true for treatments following implantations. When an implant is placed into the body, it provokes small injuries that will most of the time induce a reaction that is detrimental on the medium to long run. A way to tackle this issue is to use drugs that will locally counteract this reaction.
Drug eluting coatings have created strong interest over the recent years. They are used quite extensively today in cardiology on drug eluting stents for angioplasty and other developments are conducted in orthopedics for hip and knee implants. They can be classified into two major groups. In the first group, the drug to be eluted is mixed to the coating and will be released either in parallel to the dissolution of the coating or by diffusion through the coating or a part of the coating. In the second group, the drug is contained into the porosity of the coating that acts as a series of reservoirs. It is released as the body fluid penetrates the porosity and dissolves it.
For any type of coating, thickness is a critical aspect that has a direct impact on its stability. It is well known from the literature that thick coatings are weaker and have a higher tendency to break over time. By reducing the thickness of the coating, lifetime is improved, but as a consequence, the amount of drug that is stored is reduced. By creating small cavities that can be filled with a drug and act as reservoirs, this amount can be maintained despite the reduction in thickness.
Prior art shows different ways of creating a porous coating for drug release applications. For example Reed, Looi and Lye (CA 2 503 625) use a differential attack of a metallic alloy. By removing one component of the alloy, they create a porous layer. Brandau and Fischer (US 6,709,379) create the porosity by an electrolytic oxidation combined with an anodization. Herlein, Kovacs and Wolf (EP 1 319 416) create pores at the surface of a metallic stent through electrochemically induced pitting. These holes are then covered with a ceramic layer. In all these cases, the created porosity has the disadvantage of being homogeneous in size, or at least having a homogeneous size distribution. As a result, the loaded amount of drug will either be low (small pores) or the release will occur over a short period of time (large pores) i.e. over a few hours.
As a matter of fact, in order to store and release over a few days to a few weeks a large amount of drug, the coating must combine two porosities: one of large size acting as a reservoir and where the drug is stored and another of size similar to the released molecules that acts as a diffusion membrane.

### General description of the invention

Obtaining simultaneously a high drug loading and a slow drug release is achieved with anisotropic porous coatings having a pore size distribution in the micro or nano-size domain. These coatings are produced by the following process.
- providing a support having a surface,
- depositing on said surface at least one first mono-layer of temporary particles,
- depositing at least a coating on said temporary particles wherein said coating is porous,
- eliminating said temporary particles forming pores, to obtain a structure with a porosity with an anisotropic pore size distribution, said process furthermore comprising a coating fixation step.

Coatings obtained by the present invention are characterized by the fact that the pores are different in size and disposed in an anisotropic way. Micro-pores are created near the surface of the object and are used to store the drug, while nano-pores are disposed on the outside, towards the free surface of the coating, and act as a release membrane.

As an example, we will discuss afterwards only ceramic coatings on a metallic substrate. But the same description can be applied to any type of coating material and the coating can be made of different types of materials: metals, ceramics, polymers, hydrogels or a combination of any of these materials. It is therefore understood in that the word ceramic in the following can be replaced by polymer, metal or a combination and the same applies to metal. For differences in the process steps, table 1 describes for most of the possible combinations the modifications that need to be applied.

As the porous coating is in contact with a living body, it is preferably made of a biocompatible material. Depending on the applications this can be, but not exclusively, an oxide, a phosphate, a carbonate, a nitride or a carbonitride. Among the oxide the following ones are preferred: tantalum oxide, aluminum oxide, iridium oxide, zirconium oxide or titanium oxide. In some cases the coating can also be made of a biodegradable material that will dissolve over time and may be replaced by the living tissue. Substrates are made of materials such as metals, ceramics, polymers or a combination of any of these. Metals such as stainless steel, Nitinol, titanium, titanium alloys, or aluminum and ceramics such as zirconia, alumina, or calcium phosphate are of particular interest. It can also be a biodegradable material that will dissolve over time and may be replaced by the living tissue.

The coating has a thickness between 10 nanometers and 10 millimeters, preferably between 200 nanometers and 30 micrometers. A thicker coating allows creation of larger reservoir cavities while a thinner coating will be mechanically more resistant. The thickness is therefore chosen as an optimal value to load enough drug while maintaining perfect stability.

The porosity is created in such a way that the size distribution is anisotropic. Preferably, the median value of the pore size distribution in the coating varies from the surface of the object to the free surface of the coating, said free surface being the surface of the coating which is away from the support. Preferably, the mean value of the pore size distribution at the free surface of the coating is less than a few µm.

In another variant, the coating is made of distinct sub-layers with distinct porosity size distributions. In this case, one of the sub-layers has a mean pore size distribution of less than a few µm and preferably, the sub-layer with the smallest mean pore size distribution is located near the free surface of the coating. Preferably, both mean pore diameters differ by a factor 5 to 10.

In a preferred embodiment the smaller porosity is fixed in the nanometer range. Diffusion of a liquid through a membrane is described by the diffusion coefficient. This coefficient varies with the thickness of the membrane, the density of pores, their size as well as their tortuosity. In particular, the size of the pores will influence the diffusion if it is similar to the size of the molecule that will diffuse.

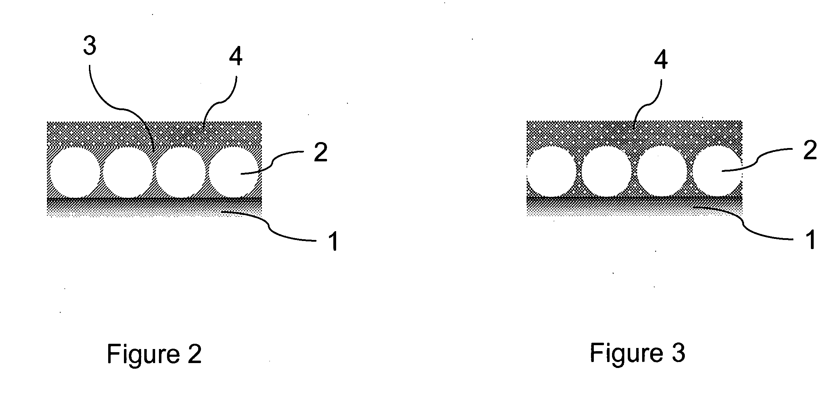

In one possible embodiment micrometer size cavities are created by depositing a template onto the implant. This template is made, for example, of mono disperse polystyrene particles that are deposited by dip coating onto the substrate. It is then partially covered with the ceramic while the diffusion membrane is produced by adding a second layer of nano-porous ceramic. Finally the template materials are removed by a thermal treatment and cavities are created. This embodiment is schematically shown in cross section in figure 2. A ceramic film made of two sublayers is coated onto a metallic substrate (1). The lower layer is made of micropores (2) (pores with diameter in the micron range) embedded in a dense ceramic (3). The upper layer is made of a nano-porous ceramic (4) (ceramic with pores in the nano-meter range).

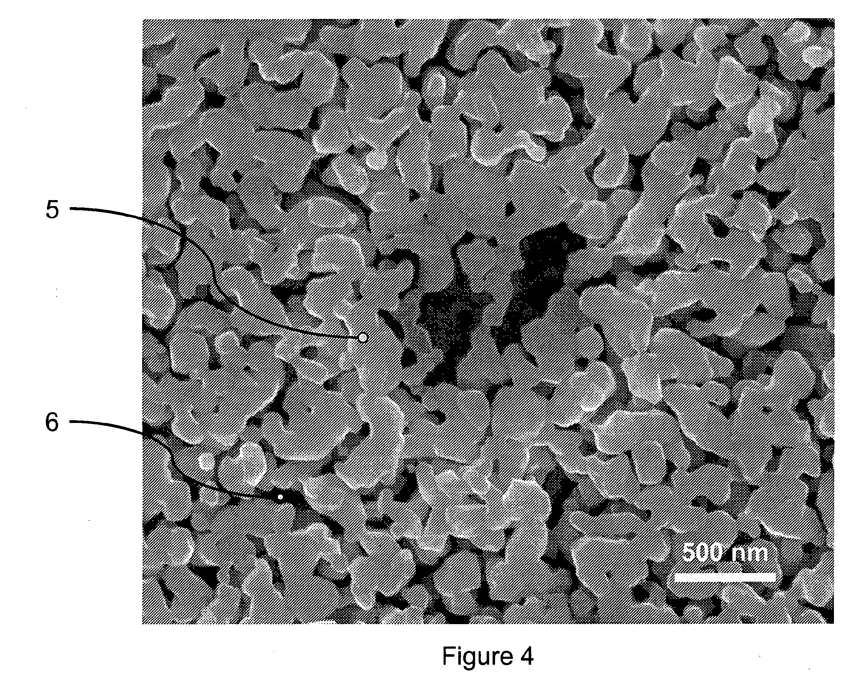

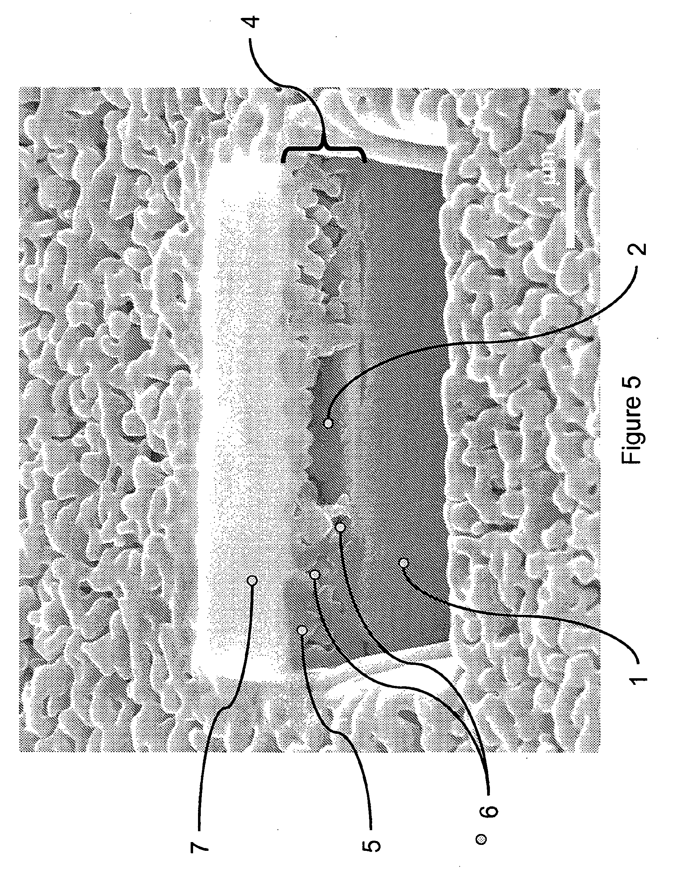

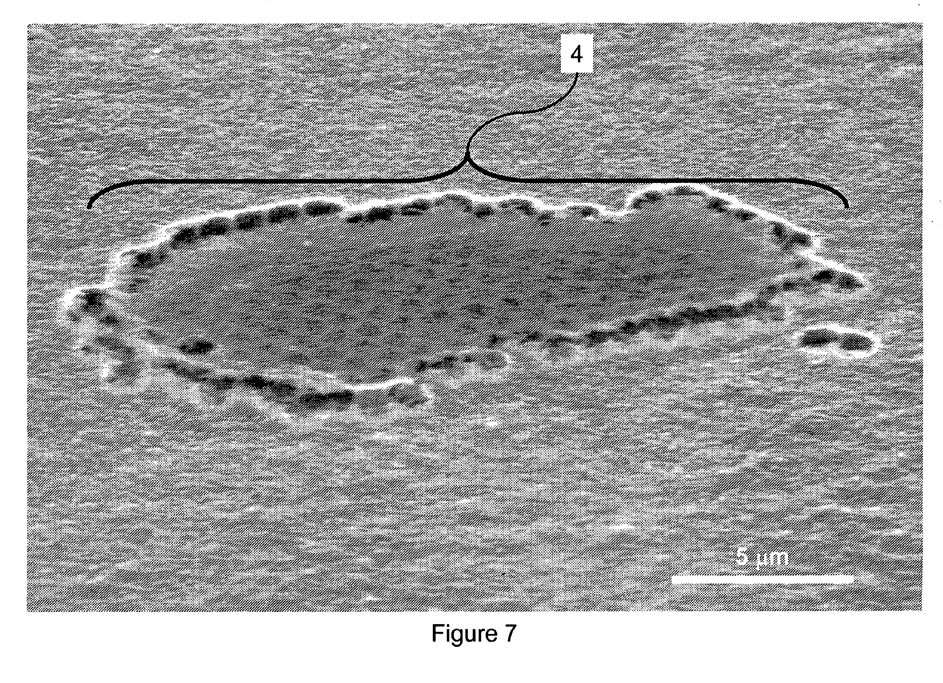

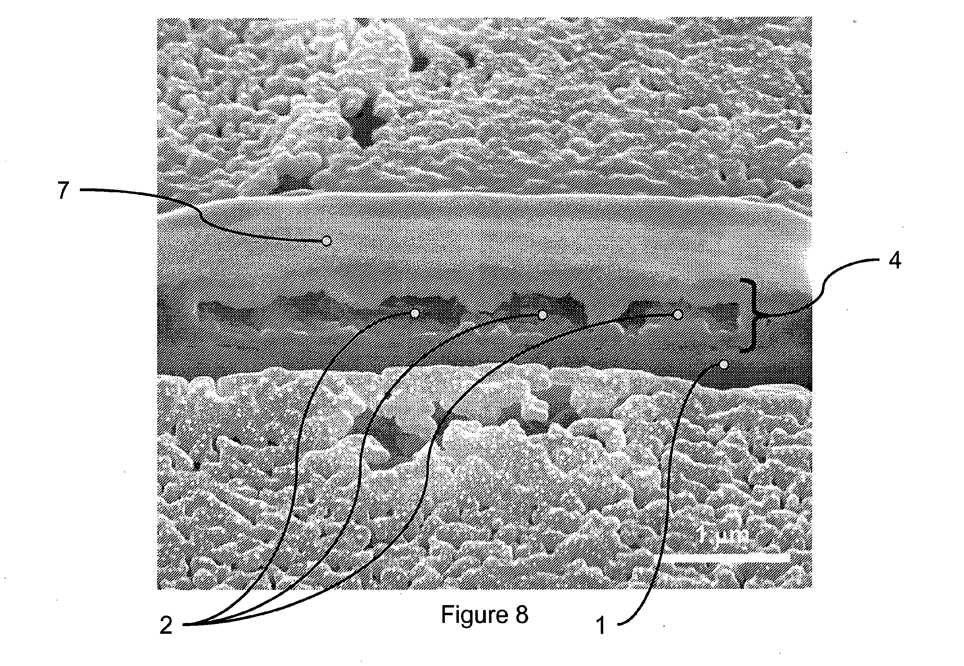

The figure 3 shows a schematic view of the cross section of another possible embodiment of the invention. In that embodiment the micro-pores (2) are embedded into the nano-porous ceramic (4). A top view photograph of this second embodiment is shown in figure 4. The micro-pore (5) with a diameter of one micrometer can be seen through the top nano-porous layer (6). The difference in contrast (black shape of the micro-pore) is due to a loading effect of the poorly conductive ceramic. The same coating is shown in figure 5 as a cross section photograph. Under a platinum bar 7 (used for sectioning the coating) the different layers of the coating can be distinguished. On the substrate (1), one has an empty pore (2) created by an eliminated temporary particles, said pore being surrounded by a porous structure (4) made of nano-particles (5) and nano-pores (6). The porosity is created in a controlled way with the larger pores located next to the substrate and the smaller pores towards the free surface of the coating. This controlled distribution creates an anisotropic porous structure in the coating. If several of these particles are placed together, one gets connected micro-cavities. Figure 7 shows a top view photograph and figure 8 a cross section photograph of such a structure.

In a further variant, the pores are made hydrophobic in order to be filled by a lipophilic solution which can be slowly released in an aqueous medium or wherein the pores are made hydrophilic in order to be filled by a hydrophilic solution.

### General coating process

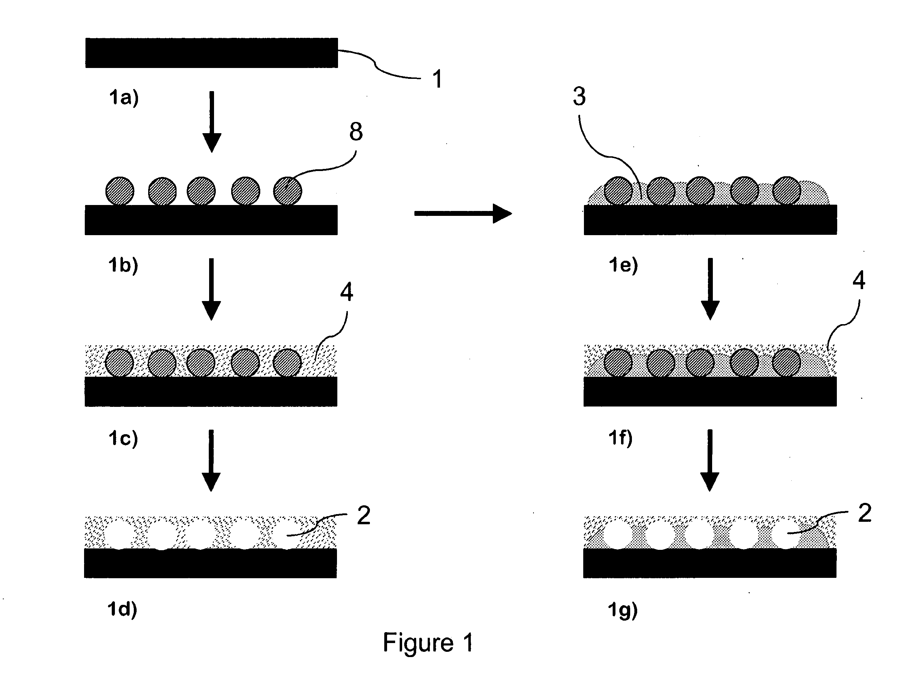

The following is a description of two possible variants of the processes used to obtain such anisotropic coatings. The steps for these variants are shown in figure 1. A first embodiment of the coating process comprises the following steps:
1) a support or substrate having a surface is provided (figure 1a));
2) one mono-layer of temporary particles is deposited onto the support or substrate to form a temporary particles construction or template (figure 1b));
3) a coating is deposited onto the support or substrate wherein the coating is nano-porous (figure 1c));
4) the temporary particles are eliminated thus forming pores in order to obtain a structure with a porosity having an anisotropic pore size distribution (figure 1d));
5) the coating is consolidated through a fixation step.

In a second embodiment of the process, the coating is made of two different coatings, a first dense coating not entirely covering said temporary particles (figure 1e)), and by a second nano-porous coating (figure 1f)), said first coating being dried before deposition of said second porous coating. Finally particles are eliminated forming pores with an anisotropic size distribution (figure 1g)) and the coating is consolidated through a fixation step. In this embodiment, the first coating forms a dense structure around the temporary particles.

In the present description, the term "eliminating" is used in a broad sense. It covers any commonly used terms related to an important change in the particle morphology, such as for example disintegration, dissolution or removal. For instance, but not exclusively, elimination of the temporary particles may comprise a thermal step, a chemical step, a mechanical step, an electro-mechanical or an irradiation step. In the case of a thermal, a chemical or an irradiation step, the temporary particles are either completely destroyed or only partially, e.g. the particles can be made hollow. In the case of a mechanical step, the temporary particles can be mechanically removed. In the case of an electro-mechanical step (e.g. sonication or ultrasonic vibrations), the particles can be swelling (e.g. by use of polymeric particles, such as PLGA) or disintegrated. More the elimination step and the consolidation step may be one single step.

The term "temporary" has to be understood as "present only for a limited time during the process". Temporary particles can be viewed as templates that create the tridimensional structure and porosity of the coating.

The expression "mono-layer of particles" means that the particles are at the same level relatively to the surface of the support. For each mono-layer, no particle will sit on top of another.

### Temporary particles deposition or templates

Preferably, in the present invention, this step is carried out by dip-coating. The temporary particles are in a solution (for example water) and the substrate is dipped in said solution. As is known in the art, the density of particles present on the substrate will depend on the concentration of particles in the solution, the rate of withdrawal of the substrate and also the surface treatment of the substrate. All these parameters may be adjusted by the skilled man to attain the desired density of particles on the substrate.

The temporary particles and the coating may be deposited together as a slurry.

### Particles

The diameter and the shape of the temporary particles can be chosen arbitrarily. But a preference is given for homogeneous particles in shape and size. The chemical composition of the particles is also free, but it is preferably selected in the group of polymers, starch, silica, metals or biological materials such as cells. A preference is given for polymers materials with a spherical shape and homogeneous diameter: mono-disperse polymer beads. For example, polystyrene bead may be advantageously used. They are readily available in numerous sizes and are very consistent in size. Alternatively, biocompatible polymers (e.g. PLGA or Poly Lactide Glycolide Acid type) can also be used.

When deposited on the support temporary particles can either be in contact with each other or separated by some empty space. When in contact, the contact surface size can be modified by changing the surface chemistry and surface affinity of the particles. It can be increase by using wettable particles or reduced to a point-like contact when using non-wettable particles such as Teflon.

Using hydrophilic and / or hydrophobic temporary particles allows the creation of various structures in the coating. Before the deposition of the temporary particles, the substrate is locally covered with a hydrophilic respectively a hydrophobic layer. In this way specific zones are adapted to fix temporary particles with a similar surface affinity while attachment on the other zones is prevented. In the case of a stent, it may be advantageous to only coat regions which are less subject to deformations; alternatively it may be advantageous to only coat regions which are in contact with the intima of the vessel to target the release of drug to prevent proliferation or inflammation. In the case of bone or dental implants, it may be advantageous to select regions where bone ingrowth should be favored and where it should be hindered.

### Coating deposition

Different procedures can be considered for the coating deposition. They are chosen according to the coating precursors that are used as well as to the desired properties of the coating. A few examples are given below:

A first procedure to deposit the coating onto the substrate uses a mixture of nanoparticles in a solvent such as for example water as coating precursor. The substrate is dipped into the precursor mixture and pulled out at a controlled speed. The thickness of the coating varies with the viscosity of the mixture and with the pulling speed.
Another procedure uses a sol obtained through hydroxylation and partial condensation of a metallic alkoxyde as coating precursor. Again, the precursor can be coated onto the substrate using either dip or spin coating.

In an other procedure, a slurry containing both the removable particles and the coating precursor dissolved in, for example, water is coated onto the substrate.

In all cases the coating can be deposited in several steps or sublayers. Between the depositions of each sub-layer the solvent of the coating precursor can be partially or fully removed by, for example, a thermal treatment. This approach permits the formation of thicker, crack-free coatings. The composition of the coating precursor can also be modified between each step. This allows the creation of coatings with variable chemical composition. For example, the chemical composition of the coating can be very similar to that of the substrate at the coating / substrate interface and can be very compatible at the interface with the body.

Using nanopowders or a sol-gel approach for producing coatings offers the advantage of reducing the necessary temperature for obtaining crystalline coatings. This is particularly favorable for metallic substrates that may go through phase transitions when thermally treated and therefore lose part of their mechanical or shape memory properties.

According to the first embodiment of the process, there is only the deposition of one single first coating entirely covering the temporary particles, said single coating having a nano-porous structure. Such porous structure thus allows the elimination of the particles as indicated here under.

According to the second embodiment of the process, the coating comprises in fact two coatings, a first coating which is treated after deposition (for example dried), said first coating not covering entirely the deposited particles, and then a second coating having a porous structure as in the first embodiment of the process.

Typically, these coatings according to the two embodiments indicated above are realized using nano-powders or by a sol-gel route, known per se in the art. Of course, other methods for creating a porous structure may be envisaged in the present invention.

### Particles removal

The elimination of the temporary particles can be achieved by different methods such as for example, but not exclusively, a thermal, a chemical, a mechanical, an electro-mechanical, a photo-chemical or an irradiation step. It can also take place at different stages of the process, before and / or during and / or after the fixation step, depending on the coating requirements

### Fixation

Any appropriate method can be used for the fixation step. Advantageously a drying step is used.

The coating fixation step can take place before the particle elimination step or it can take place simultaneously with the particle elimination step or even after the particle elimination step.

For ceramics this can be sintering where the crystalline phase is formed. For a polymer this can be a photo-chemically (by visible of UV light), a thermally or chemically induced polymerisation. For metals or for certain ceramics this can be a thermal treatment under controlled (neutral or reducing) atmosphere.

### Example .

The following example describes the realization of a coating having a structure similar to that presented in figures 3 to 6.
In one embodiment, a preferred substrate such as 316L stainless steel is used and will be coated. The substrate preparation can be electrochemically polished as described by Verma et al. (Biomed Mater Eng, 2006, 16, 381-395).
A suspension of Ti02 nanoparticles (Techpowder, Lausanne, Switzerland) is prepared with addition of PVA (Polyvinyl Alcohol), the bonding agent, and ammonia to help stabilize the colloid. The nanoparticle specifications are D10=38nm, D50=62nm, D90=82nm (CPS Disc Centrifuge^{™}).
A dip-coater (PL 3201 from Speedline Technologies^{™}) is prepared and set to the withdrawal speed of 90 [mm/sec]. The substrate is dipped first into a water based suspension of 1. micrometer diameter polystyrene microbeads (Duke Scientific, Berkeley USA). The number of beads deposited per surface area varies with their concentration in the suspension and the withdrawal speed. The substrate now covered with microbeads is then dip-coated for a second time in the same ceramic nanopowder suspension, as described previously, to cover the beads with a layer of Ti02. The ceramic layer can vary in thickness depending on the process parameters and intended use. In this example the beads will be completely covered. The Ti02 suspension will form a compact layer covering the beads. The final layer thickness is approximately 1.5 micrometers.
Finally, after a drying period of 10 minutes in a controlled climate chamber at 10% RH and 37°C, the coated substrate is sintered in an oven in a two stage process. A first bum-off step is performed at 500°C under air for 1 hour. This step will see the polymer beads burn off and leave a lenticular shaped cavity in the Ti02 layer. A second stage of sintering immediately follows the first at 800°C for 1.5 hours with a controlled Argon atmosphere. This second stage will serve to consolidate the ceramic layer.

### Using ink-jet method to create and load the coating

There are different types of ink-jet printing technologies available today. As an example we describe hereafter the drop-on-demand technology. In this technology, micro-droplets of a substance are projected through a nozzle onto a surface. The nozzle and/or the surface can be moved in all spatial directions (x,y,z). This movement allows a precise control on the final localization of the droplet on the surface. To stop the droplets and prevent them to reaching the surface, an electric field is applied at the nozzle exit that will deflect their trajectory and bring them back to the reservoir.

### Advantages of the ink-jet approach

The ink-jet method can be applied to every step of the coating deposition as described above:
- For the deposition of the template
- For the deposition of the ceramic coating
- For the filling of the pores with an active substance
For each step it allows a perfect spatial control of the process. Spatial resolution of the inkjet method is of the order of a few tenths of micrometers.

### Depositing the template with an ink-jet method

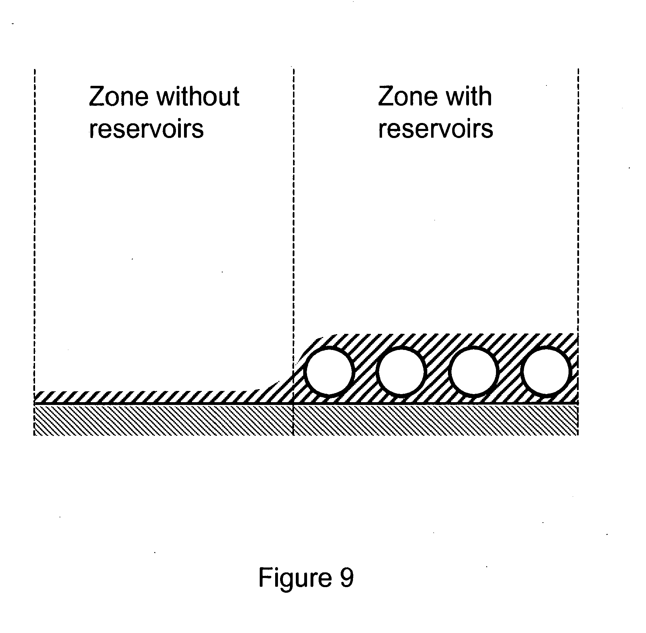

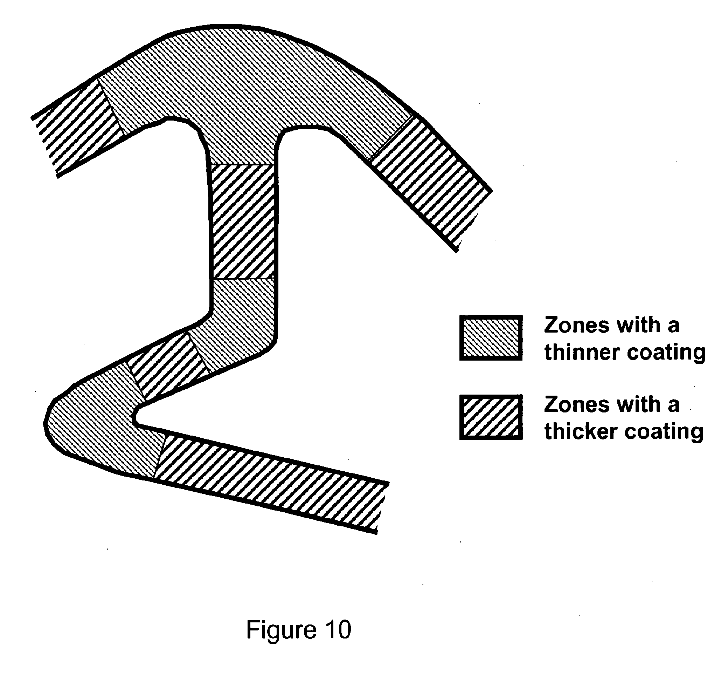

Defining zones with template particles and zones without template particles allows the creation of zones with reservoirs and zones without such reservoirs (figure 9). Zones with reservoirs will then be loaded with an active substance, while zones without reservoirs will be free of such active substance. In the case of coated stents, and in a particular embodiment, cavities will only be created towards the outside of the stent, on the surface that is in contact with the vessel wall. This geometry will minimize diffusion of drug directly into the blood stream.
Another advantage of this approach is the possibility of varying the thickness of the ceramic coating. A critical aspect of ceramic coating is their relatively low resistance to deformation. When the shape of the substrate is modified, the coating has to adapt to the new shape and this may create cracks in the layer. These cracks may generate some delamination and as a consequence particle release. Resistance to deformation can be strongly improved by using thin coatings. A zone without reservoirs will be thinner and therefore more resistant to deformation. In a particular embodiment, template beads can be deposited only in regions where deformation is low. Regions with high deformation can be coated with ceramic only, as shown in figure 10.

### Depositing the ceramic coating with an ink-jet method

Here again, the ink-jet method offers a high flexibility. Ceramic with various compositions and porosities can be coated on different parts of the substrate. For example the outside porosity of the coating (diameter and length of the pores, tortuosity) is a key element that will drive the elution profile of a loaded active substance. Having various porosities in different regions of the coating will allow the creation of different release profiles. Ink-jet therefore allows the deposition of different nanoparticle suspensions in different locations on the stent, thus leading to different outside porosities and substance release profiles.

### Filling the coating with an ink-jet method

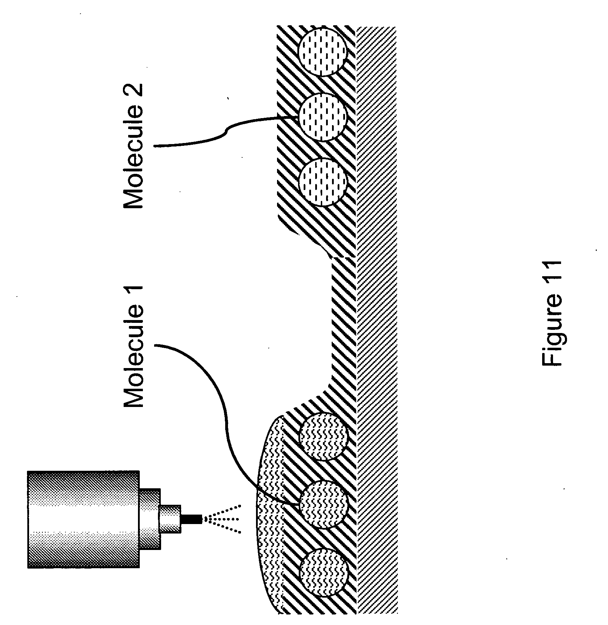

In one embodiment the coating is filled with an active substance according to the following procedure:
- The coated device is placed in a closed chamber and vacuum is applied.
- A solution containing the active substance is deposited on the surface
- Vacuum is then broken. The combination of external pressure plus vacuum in the pores will drive the solution into the pores.
- Solvent is evaporated
By using an ink-jet method to fill the pores, it is possible to specifically load certain regions with a given substance. Different molecule compositions can be loaded on a single coating (figure 11) and their spatial localization can be tuned (for example a certain type of substance can mostly be localized at the extremities of the coated device, while another substance is principally positioned in the centre).

### Object

The processes previously discussed allow the manufacturing of coatings and objects carrying such coatings with specific and original features.

### Application

A major application for these objects, as can be readily understood from the different embodiments and variants described above, is in the field of medical implants. Of particular interest are stents, orthopedic and dental implants. The porosity can be used as a drug reservoir that will release its content in a controlled way over time or it can be used to favor tissue ingrowth and therefore increase the mechanical interlocking between the implant and the living tissue.

For stents the coating can be loaded with one or several drugs. It can be a combination of the following drugs given as non-exclusive examples: an anti-proliferative agent, an anti-coagulation substance, an anti-infectious, a bacteriostatic substance.

The object can also be an orthopedic or dental implant wherein the pores may be adapted in the same manner as for the stent discussed above. In such case, the porosity obtained can either be of interest to store growth factors such as bone growth factors, increase biocompatibility or create regions where bone or cartilaginous tissue can grow and attach in a solid manner to the implant. This can also be achieved by filling the cavities with resorbable bioactive ceramics such as calcium phosphates.

Accordingly the support can be made of metal, of ceramic or polymer. It can also be made of a biodegradable material.

In this application, the coating may comprise non-porous domains. Such domains may have a minimal dimension larger than 10 micrometres and a maximal dimension smaller than 10 millimetres. In a variant, these domains have a minimal dimension larger than 100 micrometers. In another variant, these domains have a maximal dimension smaller than 1 millimetre.

The pore size may also be adapted for diffusing beads, particles or polymers containing an active substance which can be slowly released.

Alternatively the beads or particles can emit an irradiation. Advantageously, in such case, the beads or particles shall remain within the cavities.

### Short description of the figures

Figure 1 shows the different process step for two possible embodiments.
Figure 2 is a possible embodiment of the coating.
Figure 3 is another possible embodiment of the coating.
Figure 4 is a first photograph of an object which has undergone the process according to the invention.
Figure 5 shows a second photograph of an object which has undergone the process according to the invention.
Figure 6 is a third photograph of an object which has undergone the process according to the invention.
Figure 7 is a fourth photograph of an object which has undergone the process according to the invention.
Figure 8 is a fifth photograph of an object which has undergone the process according to the invention.
Figure 9 shows a schematic of regions with reservoirs and regions without.
Figure 10 shows a possible distribution of thin and thick coatings on a stent strut.
Figure 11 shows a schematic of the filling procedure.
Table 1 summarizes different possibilities for manufacturing a porous surface according to the present invention.

### Figures and tables

The present invention will be more fully understood from the following figures and table:

**Table 1**

| **Template's materials** | **Coating's material** | **Consolidation techniques** | **Elimination methods of the template: before, during or after the consolidation step** |
|---|---|---|---|
| Polymer | Polymer | UV-, Thermal - Polymerisation | *After* - Chemical selective dissolution |
| Polymer | Metal | Thermal - Annealing,... | *Before or after* - Chemical selective dissolution |
| | | | *Before or after* - UV-irradiation, oxygen plasma |
| | | | *During* - Pyrolsis |
| | | | *After* - Mechanical (ultrasonic,...) |
| Polymer | Ceramics | Thermal - Sintering | *Before* - Chemical selective dissolution |
| | | | *Before* - UV-irradiation, oxygen plasma |
| | | | *During* - Pyrolysis |
| Metal | Polymer | UV-, Thermal - Polymerisation | *After* - Chemical selective dissolution |
| Metal | Ceramics | Thermal - Sintering | *Before -* Chemical selective dissolution |
| Ceramics | Polymer | UV- Thermal - polymerisation | *After* - Chemical selective dissolution |
| | | | *After* - Mechanical (ultrasonic,...) |
| Ceramics | Metal | Thermal - Annealing, ... | *Before or after* - chemical selective dissolution |
| | | | *After*- Mechanical (ultrasonic,...) |
| Ceramics | Ceramics | Thermal - Sintering | *Before or after* - chemical selective dissolution |

In figure 1, the different steps of the coating process are represented. 1a) shows the substrate (1) before the process. The first step 1b) is the deposition of temporary particle (8) or template. As indicated in the description and in table 1, the particles can be made of several materials and can be deposited in a dispersed layer or in a dense layer. Figures 1c) and 1d) show one possible coating process corresponding to the embodiment shown in figure 3, while figure 1e) to 1g) show another coating process that corresponds to the embodiment shown in figure 2. In the first approach, a nano-porous coating (4) is deposited around and above the template particle 1b), while in the other approach, a dense layer (3) is first deposited around the template particles 1 e) without covering them and an nano-porous layer (4) is deposited above this dense layer and the template particles 1f). In both cases 1d) and 1g), the template particles are finally removed to create the micro-pores (2) used to load the drug. The coating thus forms a reservoir structure able to contain a drug for example, which can be released little by little through the porous layer.

Figure 2 is a schematic drawing of the cross section of a possible embodiment of the present invention. A ceramic film made of two sublayers is coated onto a metallic substrate (1). The lower layer is made of micro-pores (2) (pores with diameter in the micron range) embedded in a dense ceramic (3). The upper layer is made of a nano-porous ceramic (4) (ceramic with pores in the nano-meter range).

Figure 3 is another embodiment of the invention where the micro-pores (2) are embedded into the nano-porous ceramic (4), without the dense ceramic layer (3) shown in the embodiment of figure 2.

Figure 4 is picture of a top view of a structure made according to the first embodiment of the process of the present invention (1a) to 1d)). One sees a micro-pore (black circular shape) with a diameter of one micrometer covered by the nano-porous layer (5) and (6).

Figure 5 shows a cross section of the coating, underneath a platinum bar (7) (used for sectioning the coating) showing the different layers of the coating. On the substrate (1), one sees an empty micro-pore (2) left by an eliminated temporary particle, said pore being surrounded by a nano-porous structure (4) made of nano-particles (5) and nano-pores (6).

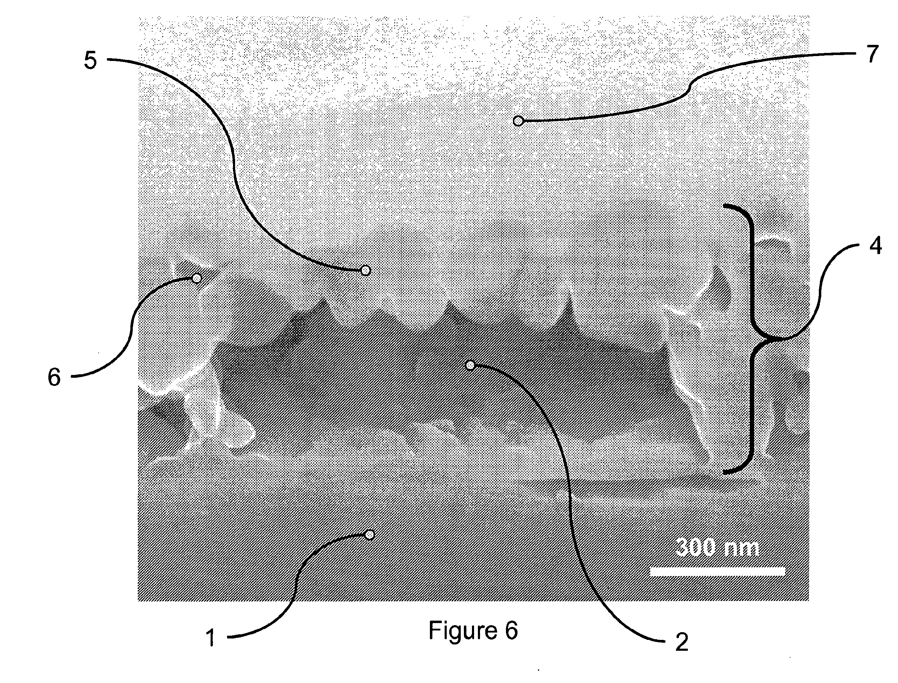

Figure 6 is a closer view of the micro-pore (2), showing the substrate (1) and the nano-porous structure (4) with the nano-particles (5), the nano-pores (6) and the platinum bar (7).

Figure 7 is a top view of the coating where a group of closely packed particles have created a series of interconnected empty micro-pores covered by a nano-porous structure (4).

Figure 8 is a cross section of the coating shown in figure 7. On the substrate (1), one sees a series of empty interconnected micro-pores (2) surrounded by a nano-porous structure (4) and covered by the platinum bar(7).

Figure 9 is a schematic drawing showing the cross section of the coating. On the left hand the coating doesn't contain any reservoirs while on right hand it contains micrometer size reservoirs.

Figure 10 is a schematic drawing of a stent strut. On this strut, portions that will undergo strong deformations are coated with a thin layer, while a thicker coating is deposited elsewhere.

Figure 11 is a schematic drawing of the filling procedure. Using the ink-jet method, a first molecule is loaded in a first region of the coating while other regions may be loaded with a second molecule.

## Claims

1. A porous coating with an anisotropic pore size distribution in the micro or nano-size domain and with a surface having a thickness between 10 nanometers and 10 millimeters, said coating being obtained by a process comprising the following steps :
- providing a support having a surface,
- depositing on said surface at least one first mono-layer of temporary particles,
- depositing at least a coating on said temporary particles wherein said coating is porous,
- eliminating said temporary particles forming pores, to obtain a structure with a porosity with an anisotropic pore size distribution, said process furthermore comprising a coating fixation step

2. A coating according to **claim 1,** wherein the median value of the pore size distribution in the coating varies from the surface of the object to the free surface of the coating.

3. A coating according to **claim 2,** wherein the median value of the pore size distribution in the coating decreases from the surface of the object to the free surface of the coating.

4. A coating according to **claim 1,** wherein the mean value of the pore size distribution at the free surface of the coating is less than 1µm.

5. A coating according to **claim 1 to 3,** wherein the coating is made of distinct sub-layers with distinct porosity size distributions.

6. A coating according to **claim 5,** wherein one of the sub-layers has a mean pore size distribution of less than 1 µm.

7. A coating according to **claim 6,** wherein the sub-layer with the smallest mean pore size distribution is located close to the free surface of the coating.

8. A coating according to **any one of the preceding claims 1 to 7,** wherein the pore sizes are adapted for storage and diffusion of an active substance for medical purposes, such as, for example a drug, an anti-coagulation substance, an anti-proliferative substance, an antibiotic substance, a bacteriostatic substance or a growth factor.

9. A coating according to **any one of the preceding claims 1 to 8,** wherein the pores are adapted to receive cells.

10. A coating according to **any one of the preceding claims 1 to 9,** wherein the coating is made of a ceramic such as an oxide (for example titanium oxide, tantalum oxide, silicon oxide, iridium oxide or zirconium oxide), a phosphate, a carbonate, a nitride or a carbonitride, or a metal, or a polymer, or an hydrogel.

11. An object with a coating as defined in **any one of the preceding claims 1 to 10.**

12. An object according to **claim 11,** wherein this object is a medical implant such as a stent or an orthopedic implant.

13. An object according to **claim 11,** wherein the support is made of metal, ceramic, polymer or any combination of those..

14. An object according to **anyone of the preceding claims 11 to 13,** wherein the coating comprises non-porous domains.

15. An object according to **claim 14,** wherein these domains have a minimal dimension larger than 10 micrometers and a maximal dimension smaller than 10 millimeters.

16. A process for manufacturing an anisotropic porous coating with a pore size distribution in the micro or nano-size domain on a support of an object and **characterized by** the following steps :
- providing a support having a surface,
- depositing on said surface at least one first mono-layer of temporary particles,
- depositing at least a coating on said temporary particles wherein said coating is porous,
- eliminating said temporary particles forming pores, to obtain a structure with a porosity with an anisotropic pore size distribution, said process furthermore comprising a coating fixation step.

17. Process according to **claim 16,** wherein said coating is made by a first layer not covering entirely said temporary particles, and by a second porous layer, said first layer being dried before deposition of said second porous layer.

18. Process according to **claims 16 to 17,** wherein said temporary particles have at least two different diameters.

19. Process according to **any one of claims 16 to 18,** wherein said temporary particles are deposited on the support in such a way as to be in contact between each other.

20. Process according to **any one of claims 16 to 19,** wherein the temporary particles and the coating are deposited together as a slurry.

21. Process according to **any one of claims 16 to 20,** wherein said temporary particles materials are selected in the group of polymers (for example polystyrene beads), starch, ceramics, silica, metals or biological material.

22. Process according to **any one of claims 16 to 21,** wherein the substrate is first partially or fully covered by a hydrophobic respectively hydrophilic layer creating hydrophobic respectively hydrophilic domains on the substrate and where hydrophobic respectively hydrophilic particles are used to build the mono-layer of temporary particles exclusively onto the hydrophobic respectively hydrophilic domains of the substrate.

23. Process according to **any one of claims 16 to 22,** wherein said temporary particles are eliminated from the layer by a thermal step, a chemical step, an electro-chemical step, a photo-chemical, a mechanical or irradiation step.

24. Process according to **any one of claims 16 to 23,** wherein said fixation step comprises a drying step.

25. Process according to **any one of claims 16 to 24**, wherein the fixation step is a temperature, UV, chemical, photo-chemical or a polycondensation step.

26. Process according to **claim 25,** wherein fixation step is followed by an anodisation step.

27. Process according to **any one of the claims 16 to 26,** wherein any of the following steps is conducted using an ink-jet method :
- temporary particles deposition
- coating deposition
- pores filling

28. Process according to **any one of the claims 16 to 27**, wherein the temporary particles are deposited on specific zones of the substrate, these zones being freely selected in advance.

29. Process according to **any one of the claims 16 to 28,** wherein the coating is deposited on specific zones of the substrate, these zones being freely selected in advance.

30. Process according to **any one of the claims 16 to 29,** wherein the filling of the pores with an active substance is done in specific zones of the substrate, these zones being freely selected in advance.

## Patentansprüche

1. Poröse Beschichtung mit einer anisotropen Porengrößenverteilung im Mikro- oder Nanometerbereich und einer Oberfläche mit einer Dicke zwischen 10 Nanometer und 10 Millimeter, wobei die Beschichtung durch ein Verfahren erhalten wird, welches folgende Schritte umfasst:
- Bereitstellen eines Trägers mit einer Oberfläche,
- Auftragen von wenigstens einer ersten Monoschicht von temporären Teilchen auf die Oberfläche,
- Auftragen von wenigstens einer Beschichtung auf die temporären Teilchen, wobei die Beschichtung porös ist,
- Entfernten der temporären Teilchen, welche Poren bilden, um eine Struktur mit einer Porosität mit einer anisotropen Porengrößenverteilung zu verhalten, wobei das Verfahren ferner einen Beschichtungs-Fixierschritt umfasst.

2. Beschichtung gemäß Anspruch 1, wobei der Medianwert der Porengrößenverteilung in der Beschichtung von der Oberfläche des Gegenstands zu der freien Oberfläche der Beschichtung variiert.

3. Beschichtung gemäß Anspruch 2, wobei der Medianwert der Porengrößenverteilung in der Beschichtung von der Oberfläche des Gegenstands zu der freien Oberfläche der Beschichtung abnimmt.

4. Beschichtung gemäß Anspruch 1, wobei der Mittelwert der Porengrößenverteilung an der freien Oberfläche der Beschichtung weniger als 1 µm beträgt.

5. Beschichtung gemäß Anspruch 1 bis 3, wobei die Beschichtung aus voneinander verschiedenen Teilschichten mit voneinander verschiedenen Porengrößenverteilungen aufgebaut ist.

6. Beschichtung gemäß Anspruch 5, wobei eine der Teilschichten eine mittlere Porengrößenverteilung von weniger als 1 µm aufweist.

7. Beschichtung gemäß Anspruch 6, wobei die Teilschicht mit der kleinsten mittleren Porengrößenverteilung nahe der freien Oberfläche der Beschichtung angeordnet ist.

8. Beschichtung gemäß einem der vorstehenden Ansprüche 1 bis 7, wobei die Porengrößen zur Speicherung und Diffusion eines Wirkstoffs für medizinische Zwecke, wie z.B. ein Arzneimittel, ein Antikoagulationsmittel, ein Antiproliferationsmittel, ein Antibiotikum, ein bakteriostatisches Mittel oder einen Wachstumsfaktor, ausgelegt sind.

9. Beschichtung gemäß einem der vorstehenden Ansprüche 1 bis 8, wobei die Poren zum Aufnehmen von Zellen ausgelegt sind.

10. Beschichtung gemäß einem der vorstehenden Ansprüche 1 bis 9, wobei die Beschichtung aus einer Keramik hergestellt, ist, wie z.B. einem Oxid (beispielsweise Titahoxid, Tantaloxid, Siliciumoxid, Iridiumoxid oder Zirkoniumoxid), einem Phosphat, einem Carbonat, einem Nitrid oder Carbonitrid, oder aus einem Metall oder einem Polymer oder einem Hydrogel.

11. Gegenstand mit einer in einem der vorstehenden Ansprüche 1 bis 10 definierten Beschichtung.

12. Gegenstand gemäß Anspruch 11, wobei der Gegenstand ein medizinisches Implantat ist, wie z.B. ein Stent oder ein orthopädische Implantat.

13. Gegenstand gemäß Anspruch 11, wobei der Träger aus Metall, Keramik, Polymer oder einer Kombination von diesen hergestellt ist.

14. Gegenstand gemäß einem der vorstehenden Ansprüche 11 bis 13, wobei die Beschichtung nichtporöse Domänen umfasst.

15. Gegenstand gemäß Anspruch 14, wobei die Domänen eine kleinste Abmessung von mehr als 10 Mikrometern und eine größte Abmessung von weniger als 10 Millimetern aufweisen.

16. Verfahren zum Herstellen einer anisotrope porösen Beschichtung mit einer Porengrößenverteilung im Mikro- oder Nanometerbereich auf einem Träger eines Gegenstands, **gekennzeichnet durch** folgende Schritte:
- Bereitstellen eines Trägers mit einer Oberfläche,
- Auftragen von wenigstens einer ersten Monoschicht von temporären Teilchen auf die Oberfläche,
- Auftragen von weinigstens einer Beschichtung auf die temporären Teilchen, wobei die Beschichtung porös ist,
- Entfernen der temporären Teilchen, welche Poren bilden, um eine Struktur mit einer Porosität mit einer anisotropen Porengrößenverteilung zu erhalten, wobei das Verfahren ferner einen Beschichtungs-Fixierschritt umfasst.

17. Verfahren gemäß Anspruch 16, wobei die Beschichtung aus einer ersten Schicht hergestellt ist, welche die temporären Teilchen nicht vollständig bedeckt, und aus einer zweiten porösen Schicht, wobei die erste Schicht vor dem Auftragen der zweiten porösen Schicht getrocknet wird.

18. Verfahren gemäß Ansprüchen 16 bis 17, wobei die temporären Teilchen wenigstens zwei unterschiedliche Durchmesser aufweisen.

19. Verfahren gemäß einem der Ansprüche 16 bis 18, wobei die temporären Teilchen auf eine solche Weise auf den Träger aufgetragen werden, dass sie miteinander in Kontakt stehen.

20. Verfahren gemäß einem der Ansprüche 16 bis 19, wobei die temporären Teilchen und die Beschichtung gemeinsam als Schlamm aufgetragen werden.

21. Verfahren gemäß einem der Ansprüche 16 bis 20, wobei die Materialien der temporären Teilchen ausgewählt sind aus der Gruppe von Polymeren (beispielsweise Polystyrolkügelchen) , Stärke, Keramik, Siliciumdioxid, Metallen und biologischem Material.

22. Verfahren gemäß einem der Ansprüche 16 bis 21, wobei das Substrat zuerst teilweise oder vollstandig mit einer hydrophoben bzw, hydrophilen Schicht bedeckt wird, um hydrophobe bzw. hydrophile Domänen auf dem Substrat zu erzeugen, und wobei hydrophobe bzw. hydrophile Teilchen, verwendet werden, um die Monoschicht von temporären Teilchen ausschließlich auf den hydrophoben bzw. hydrophilen Domänen des Substrats herzustellen.

23. Verfahren gemäß einem der Ansprüche 16 bis 22, wobei die temporären Teilchen durch einen thermischen Schritt, einen chemischen Schritt, einen elektrochemischen Schritt, einen photochemischen, einen mechanischen oder einen Bestrahlungsschritt von der Schicht entfernt werden.

24. Verfahren gemäß einem der Ansprüche 16 bis 23, wobei der Fixierschritt einen Trocknungsschritt umfasst.

25. Verfahren gemäß einem der Ansprüche 16 bis 24, wobei der Fixierschritt ein Temperatur-, UV-, chemischer, photochemischer oder Polykondensationsschritt ist.

26. Verfahren gemäß Anspruch 25, wobei der Fixierschritt von einem Anodisierungsschritt gefolgt wird.

27. Verfahren gemäß einem der Ansprüche 16 bis 26, wobei jeder der folgenden Schritte unter Verwendung eines Inkjet-Verfahrens durchgeführt wird:
- Auftragen von temporären Teilchen,
- Auftragen einer Beschichtung,
- Füllen von Poren.

28. Verfahren gemäß einem der Ansprüche 16 bis 27, wobei die temporären Teilchen auf bestimmten Bereichen des Substrats aufgetragen werden, wobei diese Bereiche zuvor frei gewährt werden.

29. Verfahren gemäß einem der Ansprüche 16 bis 28, wobei die Beschichtung auf bestimmten Bereichen des Substrats auf getragene wird, wobei diese Bereiche zuvor frei gewählt werden.

30. Verfahren gemäß einem der Ansprüche 16 bis 29, wobei das Füllen der Poren mit einem Werkstoff auf bestimmten Bereichen des Substrats durchgeführt wird, wobei diese Bereiche zuvor frei gewählt werden.

## Revendications

1. Revêtement nanoporeux avec une répartition volumétrique anisotrope des pores dans le domaine micrométrique et manométrique et avec une surface dont l'épaisseur varie entre dix nanomètres et dix millimètres, ledit revêtement étant obtenu par un procédé qui comprend les étapes suivantes :
- fourniture d'un support ayant une surface,
- dépôt sur ladite surface d'au moins une première monocouche de particules temporaires,
- dépôt d'au moins un revêtement sur lesdites particules temporaires, dans lequel ledit revêtement Est poreux,
- élimination desdites particules temporaires qui forment des pores, pour obtenir une structure avec une porosité avec une répartition volumétrique anisotrope de pores, ledit procédé comprenant en outre une étape de fixation du revêtement.

2. Revêtement selon la revendication 1, dans lequel la valeur médiane de la répartition volumétrique des pores dans le revêtement varie de la surface de l'objet à la surface libre du revêtement.

3. Revêtement selon la revendication 2, dans lequel la valeur médiane de la répartition volumétrique des pores dans le revêtement diminue de la surface de l'objet à la surface libre du revêtement.

4. Revêtement selon la revendication 1, dans lequel la valeur médiane de la répartition volumétrique des pores à la surface libre du revêtement est inférieure à 1 µm.

5. Revêtement selon les revendications 1 à 3, dans lequel le revêtement est constitué de sous-couches distinctes avec des répartitions volumétriques de porosité distinctes.

6. Revêtement selon la revendication 5, dans lequel l'une des sous-couches possède une répartition volumétrique moyenne de pores inférieure à 1 µm.

7. Revêtement selon la revendication 6, dans lequel la sous-couche avec la plus faible répartition volumétrique moyenne de pores est située à proximité de la surface libre du revêtement.

8. Revêtement selon l'une quelconque de revendications précédentes 1 à 7, dans lequel les volumes des pores sont adaptés pour le stockage et la diffusion d'une substance active à des fins médicales, comme un médicament, une substance anticoagulante, une substance antiproliférative, une substance antibiotique, une substance bactériostatique ou un facteur de croissance.

9. Revêtement selon l'une quelconque des revendications précédentes 1 à 8, dans lequel les pores sont adaptés pour recevoir des cellules.

10. Revêtement selon l'une quelconque des revendications précédentes 1 à 9, dans lequel le revêtement est constitué d'une céramique telle qu'un oxyde (par exemple, l'oxyde de titane, l'oxyde de tantale, l'oxyde de silicium, l'oxyde d'iridium ou l'oxyde zirconium), un phosphate, un carbonate, un nitrure ou un carbonitrure, ou un métal, un polymère ou un hydrogel.

11. Objet avec un revêtement tel que défini dans l'une quelconque des revendications précédentes 1 à 10.

12. Objet selon la revendication 11, dans lequel cet objet est un implant médical tel qu'une endoprothèse ou un implant orthopédique.

13. Objet selon la revendication 11, dans lequel le support est constitué d'un métal, d'une céramique, d'un polymère ou de toute combinaison de ceux-ci.

14. Objet selon l'une quelconque des revendications précédentes 11 à 13, dans lequel le revêtement comprend des domaines non poreux.

15. Objet selon la revendications 14, dans lequel ces domaines possèdent une dimension minimale supérieure à dix micromètres et une dimension maximale inférieure à dix millimètres.

16. Procédé de fabrication d'un Revêtement poreux anisotrope avec une répartition volumétrique de pores dans le domaine micrométrique ou nanométrique sur un support d'un objet et **caractérisé par** les étapes suivantes :
- fourniture d'un support ayant une surface,
- dépôt sur ladite surface d'au moins une première monocouche de particules temporaires,
- dépôt d'au moins un revêtement sur lesdites particules temporaires, dans lequel ledit revêtement est poreux,
- élimination desdites particules temporaires qui forment des pores, pour obtenir une structure avec une porosité avec une répartition volumétrique anisotrope de pores, ledit procédé comprenant en outre une étape de fixation du revêtement.

17. Procédé selon la revendication 16, dans lequel ledit revêtement est constitué d'une première couche ne couvrant pas entièrement lesdites particules temporaires, et par une seconde couche poreuse, ladite première couche étant séchée avant le dépôt de ladite seconde couche poreuse.

18. Procédé selon les revendications 16 à 17, dans lequel lesdites particules temporaires possèdent au moins deux diamètres différents.

19. Procédé selon l'une quelconque des revendications 16 à 18, dans lequel lesdites particules temporaires sont déposées sur le support de manière à être en contact entre elles.

20. Procédé selon l'une quelconque des revendications 16 à 19, dans lequel les particules temporaires et le revêtement sont déposés ensemble sous forme d'une suspension épaisse.

21. Procédé selon l'une quelconque des revendications 16 à 20, dans lequel lesdits matériaux des particules temporaires sont choisis dans le groupe constitué de polymères (par exemple, des billes de polystyrène), d'amidon, de céramiques, de silice, de métaux et de matériaux biologiques.

22. Procédé selon l'une quelconque des revendications 16 à 21, dans lequel le substrat est premièrement partiellement ou complètement recouvert par une couche hydrophile respectivement hydrophobe créant des domaines hydrophiles respectivement hydrophobes sur la substance et où des particules hydrophiles respectivement hydrophobes sont utilisées pour construire la monocouche de particules temporaires exclusivement sur les domaines hydrophiles respectivement hydrophobes du substrat.

23. Procédé selon l'une quelconque des revendications 16 à 22, dans lequel lesdites particules temporaires sont éliminées de la couche par une étape thermique, une étape chimique, une étape électrochimique, une étape photochimique, une étape mécanique ou une étape d'irradiation.

24. Procédé selon l'une quelconque des revendications 16 à 23, dans lequel ladite étape de fixation comprend une étape de séchage.

25. Procédé selon l'une quelconque des revendications 16 à 24, dans lequel l'étape de fixation est une étape de température, d'UV, chimique, photochimique ou de polycondensation.

26. Procédé selon la revendication 25, dans lequel l'étape de fixation est suivie d'une étape d'anodisation.

27. Procédé selon l'une quelconque des revendications 16 à 26, dans lequel l'une quelconque des étapes suivantes est effectuée à l'aide d'un procédé à jet d'encre :
- dépôt de particules temporaires
- dépôt de revêtement
- remplissage des pores.

28. Procédé selon l'une quelconque des revendications 16 à 27, dans lequel les particules temporaires sont déposées sur des zones spécifiques du substrat, ces zones étant librement choisies à l'avance.

29. Procédé selon l'une quelconque des revendications 16 à 28, dans lequel le revêtement est déposé sur des zones spécifiques du substrat, ces zones étant librement choisies à l'avance.

30. Procédé selon l'une quelconque des revendications 16 à 29, dans lequel le remplissage des pores avec une substance active est effectué dans des zones spécifiques du substrat, ces zones étant librement choisies à l'avance.
